# DEMANDE DE BREVET EUROPEEN

(11) **EP 4 796 080 A1**
(43) Date de publication de la demande: **26.08.2026**
(21) Numéro de dépôt: 26159907.0
(22) Date de dépôt: 20.02.2026
(51) Int. Cl.: A61F 2/08, A61B 17/06, A61B 17/04

(54) **APPAREIL DE PRÉPARATION D'UN GREFFON LIGAMENTAIRE**

(30) Priorité: 20.02.2025 FR 2501775
(71) Demandeur: FH ORTHO, 68990 Heimsbrunn (FR); Fournitures Hospitalieres Industrie, 29000 Quimper (FR)
(72) Inventeur: D'AGOSTINI, Marjorie, 68990 HEIMSBRUNN (FR); FRADIER, Emmanuel, 68990 HEIMSBRUNN (FR); VEGEHAN, Emilie, 29000 QUIMPER (FR)
(74) Mandataire: Germain Maureau

(57) **Abrégé**

L'appareil de préparation (2) comprend une base (3) et des premier et deuxième dispositifs de maintien (6, 7) configurés pour maintenir respectivement des première et deuxième portions d'extrémité (8.1, 8.2) d'un greffon ligamentaire (8). Le premier dispositif de maintien (6) comporte un élément de support (13) et un élément de maintien (14) comportant respectivement des premières zones d'appui (15) et des deuxièmes zones d'appui (16), et configurés pour occuper une configuration de support dans laquelle les premières et deuxièmes zones d'appui (15, 16) coopèrent entre elles et empêchent le passage entre elles d'une boucle d'un dispositif de suture à boucle (41) et une configuration de passage dans laquelle l'une des premières zones d'appui (15) est écartée de la deuxième zone d'appui (16) respective de manière à autoriser le passage de la boucle du dispositif de suture à boucle (41).

## Description

### Domaine technique

La présente invention concerne de manière générale un appareil de préparation d'un greffon ligamentaire.

### Etat de la technique

La présente invention s'applique en particulier à la reconstruction du ligament croisé antérieur ou postérieur de l'articulation du genou, suite à la rupture accidentelle de ce ligament. A cet effet, il est usuel de prélever un tendon sur une autre partie du corps du patient, en général le tendon droit interne ou le tendon demi tendineux, car ce prélèvement n'entraîne pas d'invalidation. Toutefois, ces tendons ne possèdent pas naturellement une résistance mécanique suffisante pour remplir la fonction de stabilisation du genou, stabilisation à laquelle participe notamment le ligament croisé antérieur. Pour conférer une résistance mécanique suffisante à ces tendons, on leur fait subir donc un certain nombre d'opérations.

Ainsi, de façon connue, les deux extrémités du tendon prélevé (d'une longueur de 20 cm environ) sont suturées l'une à l'autre de manière à obtenir un anneau fermé, puis cet anneau est tordu en forme de huit et replié sur lui-même. On obtient ainsi un greffon ligamentaire en forme d'anneau fermé à quatre brins et présentant une longueur moyenne de 4 cm environ. Les première et deuxième portions d'extrémité de ce greffon ligamentaire sont plus particulièrement destinées à être solidarisées l'une au fémur et l'autre au tibia.

Ensuite, le chirurgien positionne le greffon ligamentaire, replié en forme de huit, sur un appareil de préparation de greffon ligamentaire afin d'effectuer, préalablement à son implantation sur le patient, plusieurs étapes de préparation. Un tel appareil de préparation comprend généralement une base configurée pour reposer sur une surface de travail, et un premier dispositif de maintien et un deuxième dispositif de maintien qui sont montés sur la base à distance l'un de l'autre et qui sont configurés pour maintenir respectivement des première et deuxième portions d'extrémité d'un greffon ligamentaire et pour positionner le greffon ligamentaire dans une position de préparation.

Lorsque le greffon ligamentaire est en position de préparation (c'est-à-dire lorsque les première et deuxième portions d'extrémité du greffon ligamentaire sont maintenues en position par les premier et deuxième dispositifs de maintien), le chirurgien suture, à l'aide d'un dispositif de suture comprenant une aiguille chirurgicale et un fil de suture, les différents brins du greffon ligamentaire entre eux par exemple soit uniquement dans deux zones de suture distinctes situées respectivement au niveau des deux portions d'extrémité du greffon ligamentaire, soit dans une zone de suture s'étendant sur la majeure partie de la longueur du greffon ligamentaire.

Une telle suture peut par exemple consister à surjeter les bords longitudinaux des différents brins entre eux à l'aide du dispositif de suture. Plus particulièrement, le chirurgien suture des premiers bords longitudinaux des brins, à savoir les bords longitudinaux qui sont situés d'un premier côté du greffon ligamentaire, les uns aux autres, puis répète une telle opération de suture pour les deuxièmes bords longitudinaux des brins, à savoir les bords longitudinaux qui sont situés d'un deuxième côté du greffon ligamentaire.

Cependant, une telle technique de suture est longue et fastidieuse, ce qui complexifie la phase de préparation du greffon ligamentaire.

Une alternative connue à la technique de suture précitée consiste à suturer les brins du greffon ligamentaire, au niveau de la ou de chacune des zones de suture à réaliser, à l'aide d'un dispositif de suture à boucle (à savoir un dispositif de suture comprenant une aiguille chirurgicale et un fil de suture comprenant une première portion d'extrémité fixée à l'aiguille chirurgicale et une deuxième portion d'extrémité fixée à la première portion d'extrémité du fil de suture de manière à former une boucle). Un tel dispositif de suture à boucle permet de comprimer et de fixer entre eux les différents brins du greffon ligamentaire avec des « boucles de suture » espacées les unes des autres.

Une telle technique de suture comprend notamment les étapes suivantes :
a) fixation d'une première portion d'extrémité du greffon ligamentaire à un dispositif de maintien prévu sur un appareil de préparation de greffon ligamentaire,
b) maintien de la deuxième portion d'extrémité du greffon ligamentaire par le chirurgien, par exemple avec une pince de préhension, également nommée clamp, ou directement avec les doigts d'une main du chirurgien,
c) insertion de l'aiguille chirurgicale à travers les brins du greffon ligamentaire, par exemple à l'aide d'un ancillaire approprié manipulé avec l'autre main du chirurgien,
d) passage de la boucle du dispositif de suture autour de la deuxième portion d'extrémité du greffon ligamentaire (et le cas échéant également autour de la pince de préhension),
e) réalisation d'une traction sur l'aiguille chirurgicale et le fil de suture de manière à réaliser une « boucle de suture » autour des brins du greffon ligamentaire, et
f) répétition des étapes b) à e) pour chaque boucle de suture à réaliser sur le greffon ligamentaire.

Une telle technique de suture permet de réduire le temps de préparation du greffon ligamentaire, et plus particulièrement la durée de l'étape de suture des brins du greffon ligamentaire, et également d'assurer une meilleure compression des différents brins.

Toutefois, une telle technique de suture est malaisée en particulièrement en raison de la nécessité de devoir passer chaque « boucle de suture » autour d'une portion d'extrémité du greffon ligamentaire qui est maintenue par le chirurgien.

### Résumé de l'invention

La présente invention vise à remédier à ces inconvénients.

Le problème technique à la base de l'invention consiste donc à fournir un appareil de préparation d'un greffon ligamentaire qui soit de structure simple et économique, tout en permettant une préparation fiable, rapide et aisée d'un greffon ligamentaire.

A cet effet, la présente invention concerne un appareil de préparation d'un greffon ligamentaire, et par exemple un greffon ligamentaire en forme d'anneau, comprenant :
- une base, par exemple comportant une plaque de support, configurée pour reposer sur une surface de travail,
- un premier dispositif de maintien et un deuxième dispositif de maintien qui sont montés sur la base à distance l'un de l'autre et qui sont configurés pour maintenir respectivement des première et deuxième portions d'extrémité d'un greffon ligamentaire, et par exemple un greffon ligamentaire en forme d'anneau,
le premier dispositif de maintien comportant un élément de support qui est monté sur la base et un élément de maintien qui est configuré pour maintenir la première portion d'extrémité du greffon ligamentaire, l'élément de support et l'élément de maintien étant distincts l'un de l'autre et comportant respectivement des premières zones d'appui et des deuxièmes zones d'appui qui sont configurées pour coopérer entre elles, et l'élément de support et l'élément de maintien étant configurés pour occuper au moins une configuration de support dans laquelle l'élément de maintien est supporté par l'élément de support et dans laquelle les premières zones d'appui prévues sur l'élément de support coopèrent respectivement avec les deuxièmes zones d'appui prévues sur l'élément de maintien et empêchent le passage d'une boucle d'un dispositif de suture à boucle entre l'élément de support et l'élément de maintien et au moins une configuration de passage dans laquelle l'élément de maintien est supporté par l'élément de support et dans laquelle au moins l'une des premières zones d'appui est écartée de la deuxième zone d'appui respective de manière à autoriser le passage de la boucle du dispositif de suture à boucle entre lesdites première et deuxième zones d'appui qui sont écartées l'une de l'autre.

Une telle configuration de l'appareil de préparation selon la présente invention, et en particulier du premier dispositif de maintien, assure un maintien en position des première et deuxième portions d'extrémité d'un greffon ligamentaire à préparer, à l'aide des premier et deuxième dispositifs de maintien, lors des opérations de suture des différents brins du greffon ligamentaire, tout en permettant l'utilisation d'un dispositif de suture à boucle pour réaliser ladite opération de suture. En particulier, chaque boucle de suture peut être aisément passée autour de la première portion d'extrémité du greffon ligamentaire en déplaçant l'élément de support et l'élément de maintien dans une configuration de passage, ou successivement dans plusieurs configurations de passage différentes.

Ainsi, le chirurgien dispose de ses deux mains pour réaliser les différentes boucles de suture, et n'a notamment pas à retenir l'une des portions d'extrémité du greffon ligamentaire lors de chaque insertion de l'aiguille chirurgicale au travers des brins et n'a pas à relâcher ladite portion d'extrémité lors du passage de chaque boucle de suture autour celle-ci.

Par conséquent, l'appareil de préparation selon la présente invention facilite grandement les opérations de suture à réaliser sur un greffon ligamentaire préalablement à son implantation sur un patient.

L'appareil de préparation peut en outre présenter une ou plusieurs des caractéristiques suivantes, prises seules ou en combinaison.

Selon un mode de réalisation de l'invention, le nombre de premières zones d'appui est compris entre 3 et 10, et par exemple égal à 4, et le nombre de deuxièmes zones d'appui est compris entre 3 et 10, et par exemple égal à 4.

Selon un mode de réalisation de l'invention, les premières zones d'appui et/ou les deuxièmes zones d'appui sont arrondies.

Selon un mode de réalisation de l'invention, les premières zones d'appui ou les deuxièmes zones d'appui sont en relief, et sont par exemple formées par des ergots d'appui.

Selon un mode de réalisation de l'invention, les premières zones d'appui et les deuxièmes zones d'appui sont respectivement en relief et en creux, ou inversement. Les zones d'appui en creux peuvent par exemple être formées par des empreintes d'appui.

Selon un mode de réalisation de l'invention, l'élément de support comporte une face avant sur laquelle sont ménagées les premières zones d'appui et l'élément de maintien comporte une face arrière sur laquelle sont ménagées les deuxièmes zones d'appui, la face avant de l'élément de support et la face arrière de l'élément de maintien étant configurées pour être situées en regard l'une de l'autre et pour être décalées l'une de l'autre d'une distance d'espacement prédéterminée lorsque l'élément de support et l'élément de maintien occupent la configuration de support.

Selon un mode de réalisation de l'invention, la face avant de l'élément de support et la face arrière de l'élément de maintien sont sensiblement planes et s'étendent sensiblement parallèlement l'une par rapport à l'autre lorsque l'élément de support et l'élément de maintien occupent la configuration de support.

Selon un mode de réalisation de l'invention, la face avant de l'élément de support et la face arrière de l'élément de maintien s'étendent sensiblement verticalement lorsque l'élément de support et l'élément de maintien occupent la configuration de support et que la base repose sur une surface horizontale.

Selon un mode de réalisation de l'invention, l'appareil de préparation comporte un dispositif de fixation magnétique configuré pour fixer, de manière amovible et par attraction magnétique, l'élément de maintien à l'élément de support.

Selon un mode de réalisation de l'invention, le dispositif de fixation magnétique est configuré pour fixer l'élément de maintien à l'élément de support selon une première configuration de support dans laquelle l'élément de maintien présente une première orientation angulaire par rapport à l'élément de support, et selon une deuxième configuration de support dans laquelle l'élément de maintien présente une deuxième orientation angulaire par rapport à l'élément de support, les première et deuxième orientations angulaires étant décalées l'une par rapport à l'autre autour d'un axe de décalage qui est sensiblement horizontal lorsque la base repose sur une surface horizontale.

Selon un mode de réalisation de l'invention, les première et deuxième orientations angulaires sont décalées angulairement l'une par rapport à l'autre d'un angle de décalage d'environ 90°.

Selon un mode de réalisation de l'invention, le dispositif de fixation magnétique comprend une portion de coopération qui est en matériau ferromagnétique (et par exemple en matériau ferromagnétique dur ou en en matériau ferromagnétique doux) et qui est prévue sur l'un parmi l'élément de support et l'élément de maintien, et au moins un aimant permanent qui est prévu sur l'autre parmi l'élément de support et l'élément de maintien et qui est configuré pour coopérer par attraction magnétique avec la portion de coopération.

Selon un mode de réalisation de l'invention, le dispositif de fixation magnétique est configuré de telle sorte que, lorsque l'élément de maintien et l'élément de support occupent une configuration de passage, l'attraction magnétique entre la portion de coopération et l'au moins un aimant permanent est suffisante pour maintenir en position l'élément de maintien par rapport à l'élément de support.

Selon un mode de réalisation de l'invention, l'élément de support comporte au moins un logement de réception dans lequel est logé l'au moins un aimant permanent.

Selon un mode de réalisation de l'invention, l'au moins un logement de réception débouche dans la face avant de l'élément de support. De façon avantageuse, l'au moins un aimant permanent est situé en retrait par rapport à la face avant de l'élément de support.

Selon un mode de réalisation de l'invention, l'au moins un aimant permanent comprend une pluralité d'aimants permanents.

Selon un mode de réalisation de l'invention, l'élément de maintien comporte un corps principal configuré pour être supporté par l'élément de support, et un organe de retenue monté sur le corps principal et configuré pour maintenir la première portion d'extrémité du greffon ligamentaire.

Selon un mode de réalisation de l'invention, le corps principal comporte une partie de retenue, telle qu'une patte de retenue, et l'élément de maintien comporte un mécanisme de déplacement configuré pour déplacer l'organe de retenue par rapport au corps principal et entre une position de serrage dans laquelle l'organe de retenue et la partie de retenue sont rapprochés l'un de l'autre et sont aptes à enserrer la première portion d'extrémité du greffon ligamentaire et une position de libération dans laquelle l'organe de retenue et la portion d'appui sont éloignés l'un de l'autre et autorisent une libération de la première portion d'extrémité du greffon ligamentaire.

Selon un mode de réalisation de l'invention, le mécanisme de déplacement est configuré pour déplacer en translation l'organe de retenue par rapport au corps principal et entre la position de serrage et la position de libération.

Selon un mode de réalisation de l'invention, le mécanisme de déplacement comporte un alésage taraudé prévu sur l'organe de retenue, et une tige filetée montée mobile en rotation par rapport au corps principal et configurée pour coopérer avec l'alésage taraudé de telle sorte qu'une rotation de la tige filetée dans un premier sens de rotation entraîne un déplacement de l'organe de retenue de la position de libération vers la position de retenue, et de telle sorte qu'une rotation de la tige filetée dans un deuxième sens de rotation, opposé au premier sens de rotation, entraîne un déplacement de l'organe de retenue de la position de retenue vers la position de libération.

Selon un mode de réalisation de l'invention, l'élément de support comporte une partie de montage qui est montée, et par exemple montée coulissante, sur la base, et une partie de support s'étendant vers le haut depuis la partie de montage et apte à supporter l'élément de maintien.

Selon un mode de réalisation de l'invention, l'au moins un aimant permanent est prévu dans la partie de support.

Selon un mode de réalisation de l'invention, le premier dispositif de maintien et le deuxième dispositif de maintien sont configurés pour positionner le greffon ligamentaire, par rapport à la base, selon une direction d'extension qui est sensiblement horizontale lorsque la base repose sur une surface horizontale.

Selon un mode de réalisation de l'invention, le premier dispositif de maintien et le deuxième dispositif de maintien sont déplaçables en translation l'un par rapport à l'autre selon une direction de translation qui est sensiblement parallèle à la direction d'extension.

Selon un mode de réalisation de l'invention, le premier dispositif de maintien est monté mobile en translation par rapport à la base selon la direction de translation. De façon avantageuse, le deuxième dispositif de maintien est immobile en translation par rapport à la base.

Selon un mode de réalisation de l'invention, l'appareil de préparation comporte un organe d'immobilisation, tel qu'une vis de pression, configuré pour immobilier en position le premier dispositif de maintien par rapport à la base.

Selon un mode de réalisation de l'invention, le deuxième dispositif de maintien comporte un socle fixé à la base et un organe de maintien fixé, et par exemple fixé de manière amovible, au socle.

Selon un mode de réalisation de l'invention, le deuxième dispositif de maintien, et par exemple l'organe de maintien, comporte au moins un doigt de retenue configuré pour maintenir la deuxième portion d'extrémité du greffon ligamentaire. L'au moins un doigt de retenue peut être exemple être configuré pour s'étendre sensiblement verticalement ou sensiblement horizontalement lorsque la base repose sur une surface horizontale.

Selon un mode de réalisation de l'invention, le deuxième dispositif de maintien comporte plusieurs doigts de retenue, et par exemple plusieurs de paires de doigts de retenue, qui sont décalé(e)s selon la direction d'extension.

Selon un mode de réalisation de l'invention, l'appareil de préparation comporte un dispositif d'indication configuré pour indiquer une distance d'écartement entre une première zone de maintien, prévue sur le premier dispositif de maintien et au niveau de laquelle est apte à être maintenue la première portion d'extrémité du greffon ligamentaire, et une deuxième zone de maintien, prévue sur le deuxième dispositif de maintien et au niveau de laquelle est apte à être maintenue la deuxième portion d'extrémité du greffon ligamentaire.

Selon un mode de réalisation de l'invention, le dispositif d'indication comprend :
- des graduations portées par la base, chaque graduation correspondant à une valeur de la distance d'écartement, et
- un repère de lecture qui est associé aux graduations et qui est porté par le premier dispositif de maintien.

### Brève description des figures

La présente invention sera bien comprise à l'aide de la description qui suit en référence aux figures annexées, dans lesquelles des signes de références identiques correspondent à des éléments structurellement et/ou fonctionnellement identiques ou similaires.
Figure 1 est une vue en perspective d'un appareil de préparation d'un greffon ligamentaire selon un premier mode de réalisation de l'invention.
Figure 2 est une vue partielle en perspective de l'appareil de préparation de la figure 1.
Figure 3 est une vue partielle de dessus de l'appareil de préparation de la figure 1.
Figure 4 est une vue partielle en coupe longitudinale de l'appareil de préparation de la figure 1.
Figure 5 est une vue partielle en coupe longitudinale de l'appareil de préparation de la figure 1 selon un plan de coupe décalé latéralement par rapport à celui de la figure 4.
Figure 6 est une vue en perspective d'un élément de maintien appartenant à l'appareil de préparation de la figure 1.
Figure 7 est une vue en perspective d'un élément de support appartenant à l'appareil de préparation de la figure 1.
Figure 8, figure 9 et figure 10 sont des vues en perspective de l'appareil de préparation de la figure 1, montrant des phases successives d'une opération de suture des brins d'un greffon ligamentaire en forme d'anneau.
Figure 11 et figure 12 sont des vues en perspective d'un appareil de préparation selon un deuxième mode de réalisation de l'invention, montrant le passage d'une boucle de suture entre l'élément de support et l'élément de maintien.

### Description détaillée

Dans le présent document, les termes « inférieur », « haut » et « bas » sont définis par rapport à une utilisation de l'appareil de préparation durant laquelle la base repose sur une surface horizontale, et les termes « avant » et « arrière » sont définis par rapport à une position de préparation occupée par un greffon ligamentaire maintenu par l'appareil de préparation.

A défaut de stipulation contraire, le terme « sensiblement » signifie, dans le présent document, « exactement ou à 10% ou à 10° près ».

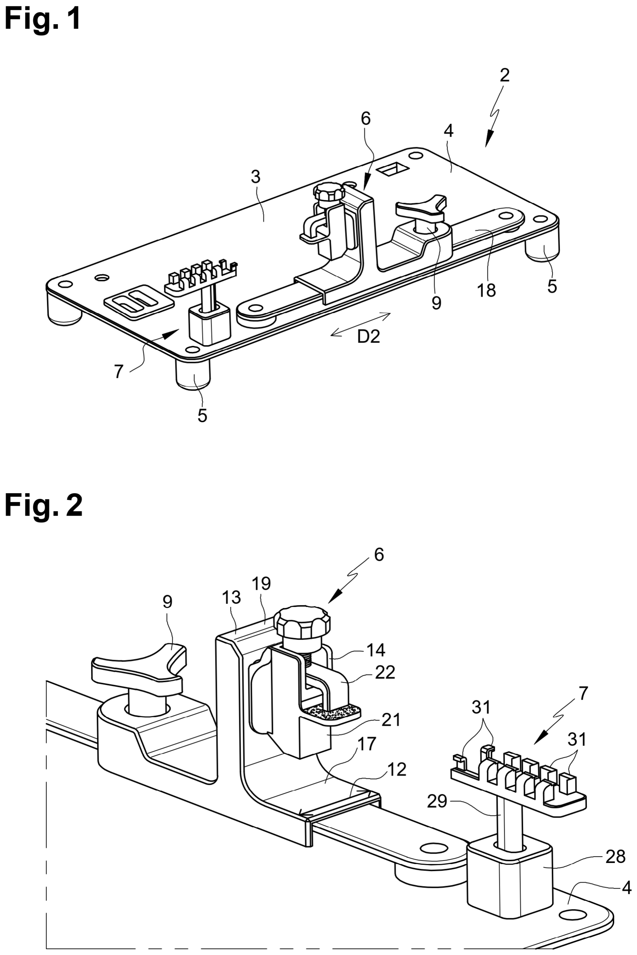

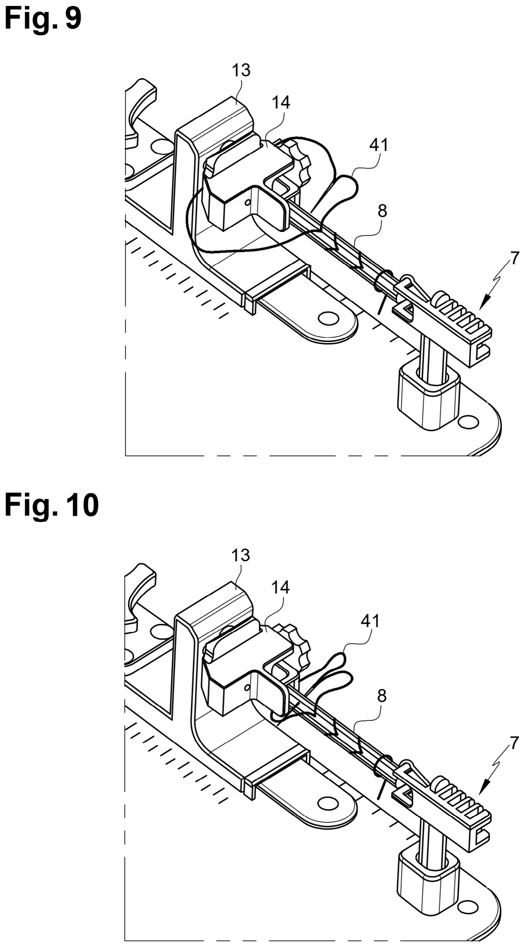

Les figures 1 à 10 représentent un appareil de préparation 2 d'un greffon ligamentaire, et plus particulièrement d'un greffon ligamentaire en forme d'anneau.

L'appareil de préparation 2 comprend notamment une base 3 configurée pour reposer sur une surface de travail. La base 3 peut par exemple comporter une plaque de support 4 équipée de plusieurs pieds 5.

L'appareil de préparation 2 comprend en outre un premier dispositif de maintien 6 et un deuxième dispositif de maintien 7 qui sont montés sur la base 3 à distance l'un de l'autre et qui sont configurés pour maintenir respectivement des première et deuxième portions d'extrémité 8.1, 8.2 d'un greffon ligamentaire 8 en forme d'anneau. Le premier dispositif de maintien 6 et le deuxième dispositif de maintien 7 sont plus particulièrement configurés pour positionner le greffon ligamentaire 8, par rapport à la base 3, selon une direction d'extension D1 qui est sensiblement horizontale lorsque la base 3 repose sur une surface horizontale.

De façon avantageuse, le premier dispositif de maintien 6 et le deuxième dispositif de maintien 7 sont déplaçables en translation l'un par rapport à l'autre selon une direction de translation D2 qui est sensiblement parallèle à la direction d'extension D1. Selon le premier mode de réalisation représenté sur les figures, le deuxième dispositif de maintien 7 est immobile en translation par rapport à la base 3, et le premier dispositif de maintien 6 est monté mobile en translation par rapport à la base 3 selon la direction de translation D2. De façon avantageuse, l'appareil de préparation 2 comporte un organe d'immobilisation 9, tel qu'une vis de pression, configuré pour immobilier en position le premier dispositif de maintien 6 par rapport à la base 3.

Selon le premier mode de réalisation représenté sur les figures, l'appareil de préparation 2 comporte un dispositif d'indication configuré pour indiquer une distance d'écartement entre une première zone de maintien, prévue sur le premier dispositif de maintien 6 et au niveau de laquelle est apte à être maintenue la première portion d'extrémité 8.1 du greffon ligamentaire 8, et une deuxième zone de maintien, prévue sur le deuxième dispositif de maintien 7 et au niveau de laquelle est apte à être maintenue la deuxième portion d'extrémité 8.2 du greffon ligamentaire 8.

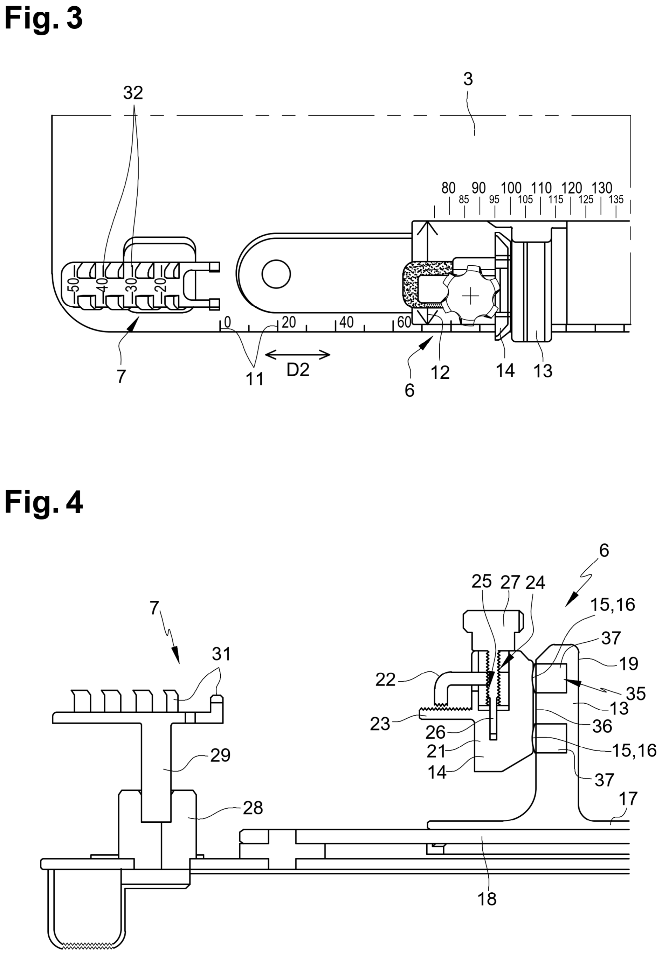

Le dispositif d'indication peut par exemple comprendre :
- des graduations 11 (voir la figure 3) portées par la base 3, chaque graduation 11 correspondant à une valeur de la distance d'écartement, et
- un repère de lecture 12 qui est associé aux graduations et qui est porté par le premier dispositif de maintien 6.

Comme montré sur la figure 2, le premier dispositif de maintien 6 comporte un élément de support 13 qui est monté mobile en translation sur la base 3 selon la direction de translation D2, et un élément de maintien 14 qui est configuré pour maintenir la première portion d'extrémité 8.1 du greffon ligamentaire 8. L'élément de support 13 et l'élément de maintien 14 sont distincts l'un de l'autre, et comportent respectivement des premières zones d'appui 15 et des deuxièmes zones d'appui 16 qui sont configurées pour coopérer entre elles, c'est-à-dire pour prendre appui respectivement les unes contre les autres, lesdites premières et deuxième zones d'appui 15, 16 étant décrites plus en détails ci-après.

Selon le premier mode de réalisation représenté sur les figures, l'élément de support 13 comporte une partie de montage 17 qui est montée, et par exemple montée coulissante, sur la base 3, et plus particulièrement sur une glissière 18 prévue sur la base 3, et une partie de support 19 s'étendant vers le haut depuis la partie de montage 17 et apte à supporter l'élément de maintien 14.

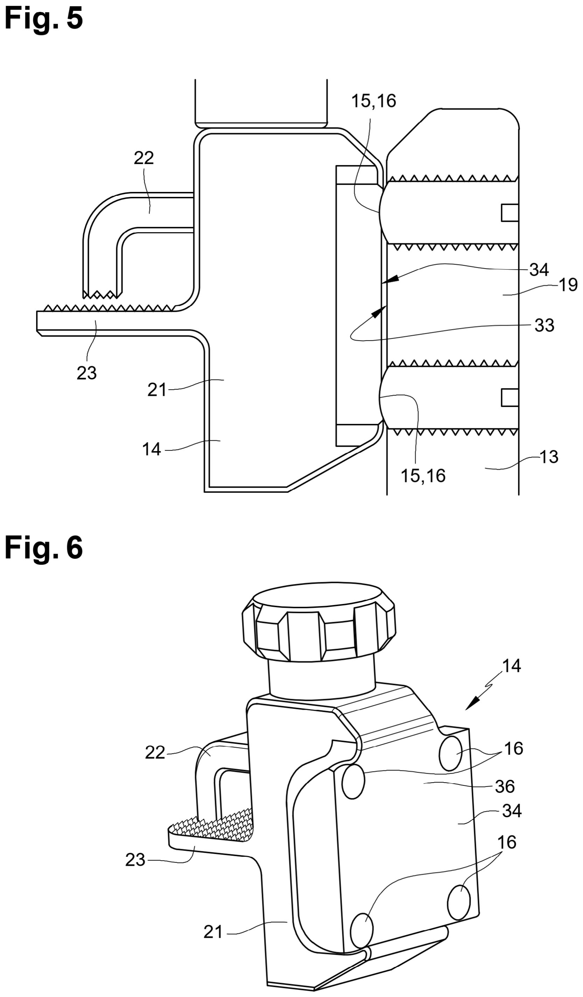

Comme montré sur la figure 6, l'élément de maintien 14 comporte un corps principal 21 configuré pour être supporté par la partie de support 19, et un organe de retenue 22 monté sur le corps principal 21 et configuré pour maintenir la première portion d'extrémité 8.1 du greffon ligamentaire 8. Selon le premier mode de réalisation représenté sur les figures, l'organe de retenue 22 est un doigt de retenue. Cependant, selon une variante de réalisation de l'invention, l'organe de retenue 22 pourrait être un crochet de retenue, une mâchoire de retenue ou encore une broche de retenue.

Selon le premier mode de réalisation représenté sur les figures, le corps principal 21 comporte une partie de retenue 23, telle qu'une patte de retenue, et l'élément de maintien 14 comporte un mécanisme de déplacement 24 configuré pour déplacer, et par exemple pour déplacement en translation, l'organe de retenue 22 par rapport au corps principal 21 et entre une position de serrage dans laquelle l'organe de retenue 22 et la partie de retenue 23 sont rapprochés l'un de l'autre et sont aptes à enserrer la première portion d'extrémité 8.1 du greffon ligamentaire 8 et une position de libération dans laquelle l'organe de retenue 22 et la portion d'appui sont éloignés l'un de l'autre et autorisent une libération de la première portion d'extrémité 8.1 du greffon ligamentaire 8.

Selon le premier mode de réalisation représenté sur les figures, l'organe de retenue 22 est bloqué en rotation par rapport au corps principal 21 et est au moins en partie monté coulissant dans le corps principal 21 (par exemple selon une direction de coulissement qui est verticale lorsque la base 3 repose sur une surface horizontale), et le mécanisme de déplacement 24 comporte un alésage taraudé 25 prévu sur l'organe de retenue 22, et une tige filetée 26 montée mobile en rotation par rapport au corps principal 21 autour d'un axe de rotation, coaxial à l'alésage taraudé 25, et configurée pour coopérer avec l'alésage taraudé 25 de telle sorte qu'une rotation de la tige filetée 26 dans un premier sens de rotation entraîne un déplacement de l'organe de retenue 22 de la position de libération vers la position de retenue, et de telle sorte qu'une rotation de la tige filetée 26 dans un deuxième sens de rotation, opposé au premier sens de rotation, entraîne un déplacement de l'organe de retenue 22 de la position de retenue vers la position de libération. De façon avantageuse, le mécanisme de déplacement 24 comporte une molette de réglage 27 solidaire en rotation de la tige filetée 26 et apte à permettre à un utilisateur d'entraîner en rotation la tige filetée 26 autour de son axe de rotation.

Selon le premier mode de réalisation représenté sur les figures, le deuxième dispositif de maintien 7 comporte un socle 28 fixé à la base 3 et un organe de maintien 29 fixé, par exemple de manière amovible, au socle 28. De façon avantageuse, l'organe de maintien 29 comporte plusieurs paires de doigts de retenue 31 qui sont décalées selon la direction d'extension D1, chaque paire de doigts de retenue 31 étant apte définir une deuxième zone de maintien pour la deuxième portion d'extrémité 8.2 du greffon ligamentaire 8. Chaque doigt de retenue 31 peut être exemple être configuré pour s'étendre sensiblement verticalement ou sensiblement horizontalement lorsque la base 3 repose sur une surface horizontale.

Lorsque l'organe de maintien 29 comporte plusieurs paires de doigts de retenue 31 qui sont décalées selon la direction d'extension D1, alors le dispositif d'indication comporte avantageusement également des graduations 32 portées par l'organe de maintien 29 et associées chacune à une paire de doigts de retenue respective, chaque graduation 32 correspondant à une valeur d'une distance de décalage entre les doigts de retenue appartenant à la paire respective et les doigts de retenue appartenant à la paire située à proximité de l'élément de support 13. Ainsi, lorsque la deuxième portion d'extrémité 8.2 du greffon ligamentaire est maintenue par des doigts de retenue 31 appartenant à la troisième paire de doigts de retenue, alors la distance séparant la deuxième zone de maintien sélectionnée et la première zone de maintien correspond à l'addition de la distance d'écartement (correspondant à la distance entre la première zone de maintien et les doigts de retenue 31 appartenant à la première paire) avec la distance de décalage.

Comme montré plus particulièrement sur la figure 5, les premières zones d'appui 15 sont en relief, et sont par exemple formées par des ergots d'appui, et les deuxièmes zones d'appui 16 sont en creux, et sont par exemple formées par des empreintes d'appui. De façon avantageuse, les premières zones d'appui 15 et les deuxièmes zones d'appui 16 sont arrondies, et sont respectivement au nombre de quatre. Toutefois, le nombre de premières zones d'appui 15, et respectivement de deuxièmes zones d'appui 16, pourrait être inférieur à quatre ou supérieure à quatre.

Selon le premier mode de réalisation représenté sur les figures, l'élément de support 13, et plus particulièrement la partie de support 19, comporte une face avant 33 qui est globalement plane et sur laquelle sont ménagées les premières zones d'appui 15, et l'élément de maintien 14, et plus particulièrement le corps principal 21, comporte une face arrière 34 qui est globalement plane et sur laquelle sont ménagées les deuxièmes zones d'appui 16. La face avant 33 et la face arrière 34 sont plus particulièrement configurées pour être situées en regard l'une de l'autre, et pour s'étendre sensiblement parallèlement l'une par rapport à l'autre lorsque l'élément de support 13 et l'élément de maintien 14 occupent une configuration de support dans laquelle les premières zones d'appui 15 sont respectivement en appui contre les deuxièmes zones d'appui 16. De façon avantageuse, la face avant 33 et la face arrière 34 sont configurées pour être décalées l'une de l'autre d'une distance d'espacement prédéterminée lorsque l'élément de support 13 et l'élément de maintien 14 occupent la configuration de support, et pour s'étendre sensiblement verticalement lorsque l'élément de support 13 et l'élément de maintien 14 occupent la configuration de support et que la base 3 repose sur une surface horizontale.

Selon le premier mode de réalisation représenté sur les figures, l'appareil de préparation 2 comporte de plus un dispositif de fixation magnétique 35 configuré pour fixer, de manière amovible et par attraction magnétique, l'élément de maintien 14 à l'élément de support 13.

Le dispositif de fixation magnétique 35 comprend plus particulièrement une portion de coopération 36 qui est en matériau ferromagnétique (et par exemple en matériau ferromagnétique dur ou en en matériau ferromagnétique doux) et qui est prévue sur l'un parmi l'élément de support 13 et l'élément de maintien 14, et un ou plusieurs aimant(s) permanent(s) 37 qui est(sont) prévu(s) sur l'autre parmi l'élément de support 13 et l'élément de maintien 14 et qui est(sont) configuré(s) pour coopérer par attraction magnétique avec la portion de coopération 36.

Selon le premier mode de réalisation représenté sur les figures, la portion de coopération 36 est formée par l'élément de maintien 14, et plus particulièrement par le corps principal 21 qui est intégralement réalisé en un matériau ferromagnétique, et le ou les aimant(s) permanent(s) 37 est(sont) prévu(s) sur l'élément de support 13. A cet effet, l'élément de support 13, et plus particulièrement la partie de support 19, peut par exemple comporter un ou plusieurs logement(s) de réception 38 dans lequel ou dans chacun desquels est logé un aimant permanent 37. De façon avantageuse, le ou chaque logement de réception 38 débouche dans la face avant 33 prévue sur l'élément de support 13, et le ou chaque aimant permanent 37 est situé en retrait par rapport à la face avant 33 de l'élément de support 13 ou est affleurant avec ladite face avant 33.

L'élément de support 13 et l'élément de maintien 14 sont plus particulièrement configurés pour occuper une configuration de support dans laquelle l'élément de maintien 14 est supporté par l'élément de support 13, via le dispositif de fixation magnétique 35, et dans laquelle les premières zones d'appui 15 prévues sur l'élément de support 13 coopèrent respectivement avec les deuxièmes zones d'appui 16 prévues sur l'élément de maintien 14 et empêchent le passage d'une boucle d'un dispositif de suture à boucle entre l'élément de support 13 et l'élément de maintien 14 et plusieurs configurations de passage dans chacune desquelles l'élément de maintien 14 est supporté par l'élément de support 13, via le dispositif de fixation magnétique 35, et dans chacune desquelles l'une des premières zones d'appui 15 est écartée de la deuxième zone d'appui 16 respective de manière à autoriser le passage d'une boucle du dispositif de suture à boucle entre lesdites première et deuxième zones d'appui qui sont écartées l'une de l'autre.

Une telle configuration de l'appareil de préparation 2 selon la présente invention, et en particulier du premier dispositif de maintien 6, assure un maintien en position des première et deuxième portions d'extrémité 8.1, 8.2, du greffon ligamentaire 8 à préparer, à l'aide des premier et deuxième dispositifs de maintien 6, 7, lors des opérations de suture des différents brins du greffon ligamentaire 8, tout en permettant l'utilisation d'un dispositif de suture à boucle pour réaliser lesdites sutures. En particulier, chaque boucle de suture peut être aisément passée autour de la première portion d'extrémité 8.1 du greffon ligamentaire 8 en déplaçant préalablement l'élément de support 13 et l'élément de maintien 14 successivement dans plusieurs configurations de passage différentes.

Ainsi, le chirurgien dispose de ses deux mains pour réaliser les différentes boucles de suture, et n'a notamment pas à retenir l'une des portions d'extrémité du greffon ligamentaire 8 lors de chaque insertion de l'aiguille chirurgicale d'un dispositif de suture à boucle au travers des brins d'un greffon ligamentaire et n'a pas à relâcher ladite portion d'extrémité lors du passage de chaque boucle de suture autour celle-ci.

Par conséquent, l'appareil de préparation 2 selon la présente invention facilite grandement les opérations de suture à réaliser sur un greffon ligamentaire 8 préalablement à son implantation sur un patient.

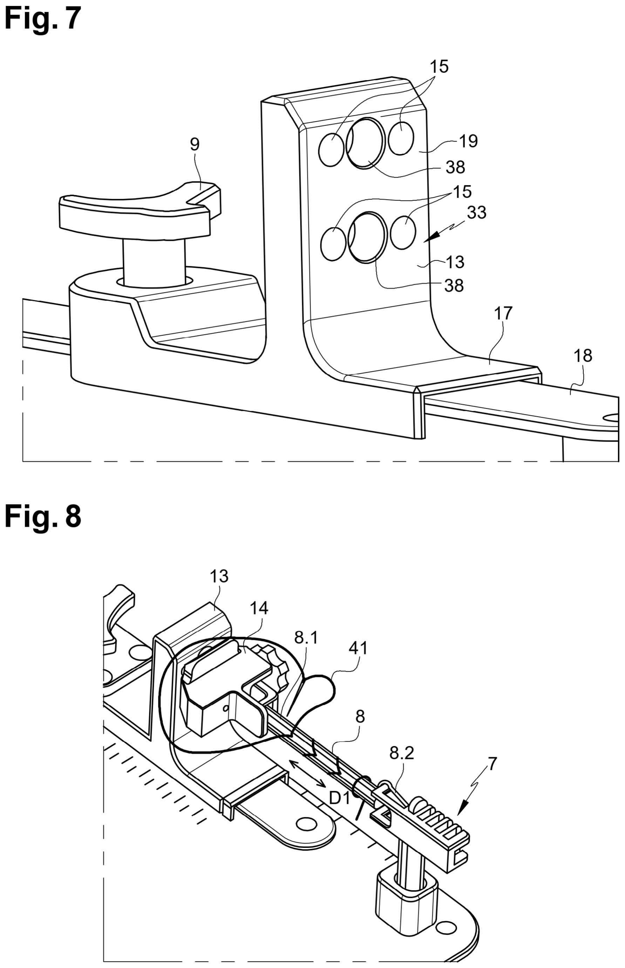

Un exemple de procédé de préparation d'un greffon ligamentaire 8 en forme d'anneau à l'aide de l'appareil de préparation 2 selon le premier mode de réalisation de l'invention est décrit ci-après en référence notamment aux figures 8 à 10.

Un tel procédé de préparation comprend les étapes suivantes :
a) fixation d'une première portion d'extrémité 8.1 du greffon ligamentaire 8 au premier dispositif de maintien 6,
b) fixation d'une deuxième portion d'extrémité 8.2 du greffon ligamentaire 8 au deuxième dispositif de maintien 7,
c) passage de la boucle d'un dispositif de suture à boucle 41 entre l'élément de support 13 et l'élément de maintien 14, en déplaçant successivement ladite boucle entre les premières zones d'appui 15 et les deuxièmes zones d'appui 16 (par exemple en tenant fermement, avec les deux mains, deux portions de la boucle situées de part et d'autre de l'élément de maintien 14, en introduisant la portion de boucle, située entre les deux mains du chirurgien, entre la face avant 33 et la face arrière 34, et en déplaçant vers le bas ladite portion de boucle jusqu'à ce qu'elle atteigne une face inférieure du corps principal 21), chaque passage de la portion de boucle entre une première zone d'appui 15 et la deuxième zone d'appui 16 respective provoquant un déplacement de l'élément de support 13 et de l'élément de maintien 14 dans une configuration de passage et ce à l'encontre de l'attraction magnétique exercée par le dispositif de fixation magnétique 35,
d) insertion de l'aiguille chirurgicale à travers les brins du greffon ligamentaire 8, par exemple à l'aide d'un ancillaire approprié,
e) réalisation d'une traction sur l'aiguille chirurgicale et le fil de suture de manière à serrer une « boucle de suture » autour des brins du greffon ligamentaire 8, et
f) répétition des étapes c) à e) pour chaque boucle de suture à réaliser sur le greffon ligamentaire 8.

Selon un mode de réalisation de l'invention, le dispositif de fixation magnétique 35 pourrait être configuré pour fixer l'élément de maintien 14 à l'élément de support 13 selon une première configuration de support (voir la figure 2) dans laquelle l'élément de maintien 14 présente une première orientation angulaire par rapport à l'élément de support 13, et selon une deuxième configuration de support (voir la figure 8) dans laquelle l'élément de maintien 14 présente une deuxième orientation angulaire par rapport à l'élément de support 13, les première et deuxième orientations angulaires étant décalées l'une par rapport à l'autre autour d'un axe de décalage qui est sensiblement horizontal lorsque la base 3 repose sur une surface horizontale. De façon avantageuse, les première et deuxième orientations angulaires sont décalées angulairement l'une par rapport à l'autre d'un angle de décalage d'environ 90°. Une telle configuration du dispositif de fixation magnétique 35 permet à un chirurgien de positionner l'organe de retenue 22 par exemple dans une première position angulaire dans laquelle ce dernier est orienté verticalement ou dans une deuxième position angulaire dans laquelle ce dernier est orienté horizontalement. Selon un tel mode de réalisation de l'invention, l'appareil de préparation 2 est avantageusement équipé deux organes de maintien 29 différents, l'un équipé de doigts de retenue 31 aptes à s'étendre verticalement et l'autre équipé de doigts de retenue 31 aptes à s'étendre horizontalement.

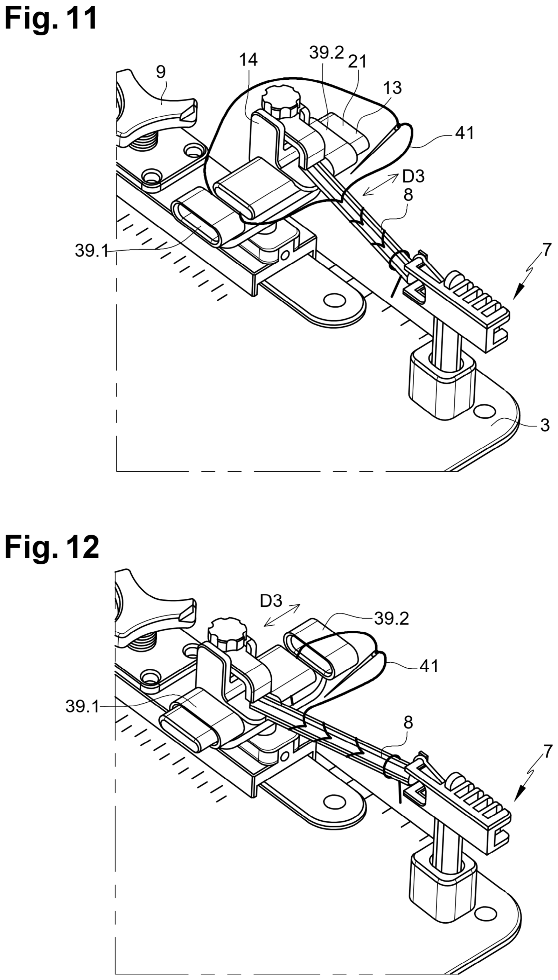

Les figures 11 et 12 représentent un appareil de préparation 2 selon un deuxième mode de réalisation de l'invention qui diffère du premier mode de réalisation décrit précédemment essentiellement en ce que l'élément de maintien 14 est monté coulissant par rapport à l'élément de support 13 selon une direction de coulissement D3 qui s'étend perpendiculairement à la direction d'extension D1 et qui est sensiblement horizontale lorsque la base 3 repose sur une surface horizontale, en ce que le corps principal 21 est allongé et s'étend selon la direction de coulissement D3 et en ce que l'élément de support 13 comporte une première partie de support 39.1 une deuxième partie de support 39.2 situées de part et d'autre de l'élément de maintien 14 et configurées pour supporter respectivement des première et deuxième portions d'extrémité opposées du corps principal 21.

Chacune des première et deuxième parties de support 39.1, 39.2 comporte plus particulièrement une première zone d'appui 15, par exemple annulaire, apte à coopérer avec une deuxième zone d'appui 16, par exemple annulaire, prévue sur la portion d'extrémité respective du corps principal 21, et l'élément de support 13 et l'élément de maintien 14 sont configurés pour occuper une configuration de support dans laquelle les première et deuxième portions d'extrémité du corps principal 21 sont respectivement reçues dans les première et deuxième parties de support 39.1, 39.2 et dans laquelle les premières zones d'appui 15 coopèrent respectivement avec les deuxièmes zones d'appui 16, une première configuration de passage dans laquelle la première portion d'extrémité du corps principal 21 est reçue dans la première partie de support 39.1 et la deuxième portion d'extrémité du corps principal 21 est située à distance de la deuxième partie de support 39.2 (et donc de la première zone d'appui 15 prévue la deuxième partie de support 39.2) de manière à définir un premier passage pour la boucle d'un dispositif de suture à boucle 41 et une deuxième configuration de passage dans laquelle la deuxième portion d'extrémité du corps principal 21 est reçue dans la deuxième partie de support 39.2 et la première portion d'extrémité du corps principal 21 est située à distance de la première partie de support 39.1 (et donc de la première zone d'appui 15 prévue la première partie de support 39.1) de manière à définir un deuxième passage pour la boucle du dispositif de suture à boucle 41.

Une telle configuration de l'appareil de préparation 2 permet un passage de la boucle d'un dispositif de suture à boucle 41 autour d'une portion d'extrémité d'un greffon ligamentaire 8 tout simplement en déplaçant l'élément de maintien 14 de la première configuration de passage à la deuxième configuration de passage, ou inversement.

Bien entendu, la présente invention n'est nullement limitée aux modes de réalisation décrits et illustrés qui n'ont été donnés qu'à titre d'exemples. Des modifications restent possibles, notamment du point de vue de la constitution des divers éléments ou par substitution d'équivalents techniques, sans sortir pour autant du domaine de protection de l'invention.

## Revendications

1. Appareil de préparation (2) d'un greffon ligamentaire (8), comprenant :
- une base (3) configurée pour reposer sur une surface de travail,
- un premier dispositif de maintien (6) et un deuxième dispositif de maintien (7) qui sont montés sur la base (3) à distance l'un de l'autre et qui sont configurés pour maintenir respectivement des première et deuxième portions d'extrémité (8.1, 8.2) d'un greffon ligamentaire (8),
**caractérisé en ce que** le premier dispositif de maintien (6) comporte un élément de support (13) qui est monté sur la base (3) et un élément de maintien (14) qui est configuré pour maintenir la première portion d'extrémité (8.1) du greffon ligamentaire (8), l'élément de support (13) et l'élément de maintien (14) étant distincts l'un de l'autre et comportant respectivement des premières zones d'appui (15) et des deuxièmes zones d'appui (16) qui sont configurées pour coopérer entre elles, et **en ce que** l'élément de support (13) et l'élément de maintien (14) sont configurés pour occuper au moins une configuration de support dans laquelle l'élément de maintien (14) est supporté par l'élément de support (13) et dans laquelle les premières zones d'appui (15) prévues sur l'élément de support (13) coopèrent respectivement avec les deuxièmes zones d'appui (16) prévues sur l'élément de maintien (14) et empêchent le passage d'une boucle d'un dispositif de suture à boucle (41) entre l'élément de support (13) et l'élément de maintien (14) et au moins une configuration de passage dans laquelle l'élément de maintien (14) est supporté par l'élément de support (13) et dans laquelle au moins l'une des premières zones d'appui (15) est écartée de la deuxième zone d'appui (16) respective de manière à autoriser le passage de la boucle du dispositif de suture à boucle (41) entre lesdites première et deuxième zones d'appui qui sont écartées l'une de l'autre.

2. Appareil de préparation (2) selon la revendication 1, dans lequel les premières zones d'appui (15) ou les deuxièmes zones d'appui (16) sont en relief.

3. Appareil de préparation (2) selon la revendication 1 ou 2, dans lequel l'élément de support (13) comporte une face avant (33) sur laquelle sont ménagées les premières zones d'appui (15) et l'élément de maintien (14) comporte une face arrière (34) sur laquelle sont ménagées les deuxièmes zones d'appui (16), la face avant (33) de l'élément de support (13) et la face arrière (34) de l'élément de maintien (14) étant configurées pour être situées en regard l'une de l'autre et pour être décalées l'une de l'autre d'une distance d'espacement prédéterminée lorsque l'élément de support (13) et l'élément de maintien (14) occupent la configuration de support.

4. Appareil de préparation (2) selon la revendication 3, dans lequel la face avant (33) de l'élément de support (13) et la face arrière (34) de l'élément de maintien (14) sont sensiblement planes et s'étendent sensiblement parallèlement l'une par rapport à l'autre lorsque l'élément de support (13) et l'élément de maintien (14) occupent la configuration de support.

5. Appareil de préparation (2) selon la revendication 3 ou 4, dans lequel la face avant (33) de l'élément de support (13) et la face arrière (34) de l'élément de maintien (14) s'étendent sensiblement verticalement lorsque l'élément de support (13) et l'élément de maintien (14) occupent la configuration de support et que la base (3) repose sur une surface horizontale.

6. Appareil de préparation (2) selon l'une quelconque des revendications 1 à 5, lequel comporte un dispositif de fixation magnétique (35) configuré pour fixer, de manière amovible et par attraction magnétique, l'élément de maintien (14) à l'élément de support (13).

7. Appareil de préparation (2) selon la revendication 6, dans lequel le dispositif de fixation magnétique (35) comprend une portion de coopération (36) qui est en matériau ferromagnétique et qui est prévue sur l'un parmi l'élément de support (13) et l'élément de maintien (14), et au moins un aimant permanent (37) qui est prévu sur l'autre parmi l'élément de support (13) et l'élément de maintien (14) et qui est configuré pour coopérer par attraction magnétique avec la portion de coopération (36).

8. Appareil de préparation (2) selon la revendication 7, dans lequel l'élément de support (13) comporte au moins un logement de réception (38) dans lequel est logé l'au moins un aimant permanent (37).

9. Appareil de préparation (2) selon l'une quelconque des revendications 1 à 8, dans lequel l'élément de maintien (14) comporte un corps principal (21) configuré pour être supporté par l'élément de support (13), et un organe de retenue (22) monté sur le corps principal (21) et configuré pour maintenir la première portion d'extrémité (8.1) du greffon ligamentaire (8).

10. Appareil de préparation (2) la revendication 9, dans lequel le corps principal (21) comporte une partie de retenue (23) et l'élément de maintien (14) comporte un mécanisme de déplacement (24) configuré pour déplacer l'organe de retenue (22) par rapport au corps principal (21) et entre une position de serrage dans laquelle l'organe de retenue (22) et la partie de retenue (23) sont rapprochés l'un de l'autre et sont aptes à enserrer la première portion d'extrémité (8.1) du greffon ligamentaire (8) et une position de libération dans laquelle l'organe de retenue (22) et la portion d'appui sont éloignés l'un de l'autre et autorisent une libération de la première portion d'extrémité (8.1) du greffon ligamentaire (8).

11. Appareil de préparation (2) selon l'une quelconque des revendications 1 à 10, dans lequel le premier dispositif de maintien (6) et le deuxième dispositif de maintien (7) sont configurés pour positionner le greffon ligamentaire (8), par rapport à la base (3), selon une direction d'extension (D1) qui est sensiblement horizontale lorsque la base (3) repose sur une surface horizontale.

12. Appareil de préparation (2) selon la revendication 11, dans lequel le premier dispositif de maintien (6) et le deuxième dispositif de maintien (7) sont déplaçables en translation l'un par rapport à l'autre selon une direction de translation (D2) qui est sensiblement parallèle à la direction d'extension (D1).
